# EUROPÄISCHE PATENTSCHRIFT

(11) **EP 1 929 024 B1**
(45) Veröffentlichungstag und Bekanntmachung des Hinweises auf die Patenterteilung: **20.07.2011**
(21) Anmeldenummer: 06805733.0
(22) Anmeldetag: 15.09.2006
(51) Int. Cl.: C12P 5/02

(54) **VERFAHREN ZUR HERSTELLUNG VON BIOGAS UNTER VERWENDUNG EINES SUBSTRATS MIT HOHEM FESTSTOFF- UND STICKSTOFFANTEIL**
PROCESS FOR THE PRODUCTION OF BIOGAS EMPLOYING A SUBSTRATE HAVING A HIGH SOLIDS AND HIGH NITROGEN CONTENT
PROCÉDÉ DE PRODUCTION DE BIOGAZ UTILISANT UN SUBSTRAT À HAUTE TENEUR EN SOLIDES ET EN AZOTE

(30) Priorität: 30.09.2005 DE 102005047719
(43) Veröffentlichungstag der Anmeldung: 11.06.2008
(73) Patentinhaber: KWK GBR, 04299 Leipzig (DE)
(72) Erfinder: WEIDELE, Thomas, 04275 Leipzig (DE)
(74) Vertreter: Nenning, Peter
(86) Internationale Anmeldenummer: PCT/EP2006/008995
(87) Internationale Veröffentlichungsnummer: WO 2007/039067

(56) Entgegenhaltungen:
- WO-A-80/01922
- WO-A-03/042117
- DE-A1- 19 547 320

## Beschreibung

Die Erfindung betrifft ein Verfahren zur energetischen Verwertung von Biomasse mit hohen Feststoff- und Stickstoffanteilen als Substrat in Biogasanlagen.

Tabelle 1 zeigt beispielhaft einige dieser Substrate. Weitere Substrate mit hohem Feststoffanteil sind u. a. Grünschnitt aller Art sowie Garten- und Lebensmittelabfälle. Weiterhin gibt es Substrate mit sehr hohem Stickstoffanteil wie Ölschrote und Schlempen. Substrate mit hohen Fremdstoffanteilen sind z. B. Hühnermist mit einem hohen Kalk-/Sand-Anteil.

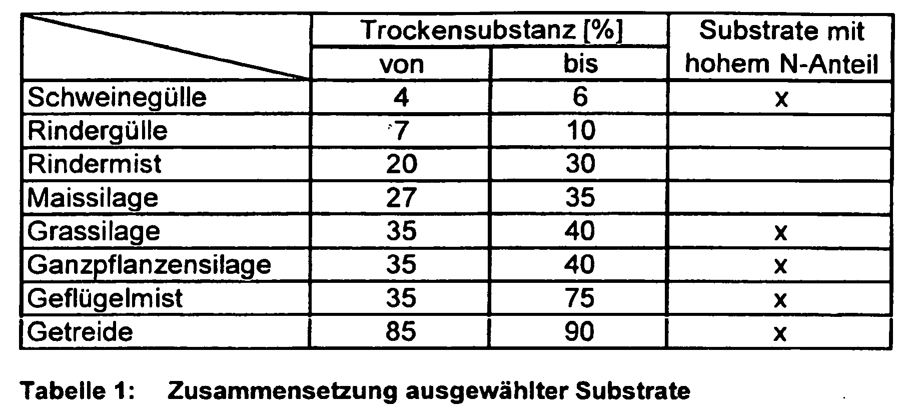

Die bekannten Biogasverfahren werden allgemein in Trocken- und Nassvergärungsverfahren unterschieden. Grundlage für einen vorteilhaften Biogasprozess ist u. a. ein optimaler Aufschluss des eingesetzten Substrates, um den verwendeten Bakterien eine möglichst große Angriffsfläche zu erschließen. Von besonderer Bedeutung sind die Umgebungsbedingungen für die Bakterien. Diese erfordern eine gleichmäßige Temperierung des Prozesses und ein Trägermedium, welches eine hohe Mobilität der Bakterien erlaubt bzw. die Zufuhr der Biomasse zu den Bakterien gewährleistet. Die Temperierung ist aufwendig, die in engen Grenzen definierte notwendige Kontaktierung zwischen Bakterien und Substrat kostenintensiv.

Bei Trockenvergärungsverfahren wird das Substrat in der Regel zerkleinert und in fester Form in ein luftdichtes Behältnis eingeschlossen, in welchem der Biogasprozess abläuft. Nachteilig bei diesen Verfahren ist der in der Regel niedrige Biogasertrag, der seine Ursachen in dem ungleichmäßigen Wärmeeintrag und damit der ungleichmäßigen Temperatur und der geringen Mobilität der Bakterien hat.

Diese wesentlichen Nachteile sind bei der Nassvergärung nicht vorhanden. Durch das flüssige Trägermedium sind sowohl eine gleichmäßige Prozesstemperatur als auch die Mobilität der Bakterien bzw. die Zufuhr des Substrates gewährleistet.

Von Nachteil bei der Nassvergärung ist jedoch, dass Substrate mit hohen Trockensubstanzanteilen laufend mit Flüssigkeit angemaischt werden müssen. Dies führt zu einer Erhöhung der Gärrestemenge mit entsprechendem logistischem Aufwand bei der Lagerung und dem Transport. Eine Möglichkeit, den Flüssigkeitsbedarf zu verringern, bietet die Rezirkulierung von Gärrest zum Anmaischen. Diese Lösung funktioniert aber nicht beim Einsatz von Substraten mit hohen Stickstoffkonzentrationen. Zur Verringerung der Stickstoffkonzentrationen bleibt nur die Möglichkeit der Verdünnung mit Flüssigkeit.

Steht zum Anmaischen nur Wasser und nicht Gülle zur Verfügung, besteht für den Prozess weiterhin die Gefahr, dass die erforderlichen Bakterien aus dem Prozess geschwemmt werden und es so zu einem Abbruch der Biogasproduktion kommt. Das mögliche Ausschwemmen der Bakterien aus dem Biogasprozess hat seine Ursache in der langen Generationszeit der Bakterien, d. h. dass mit dem Gärrest mehr Bakterien den Fermenter verlassen, als neu gebildet werden. Dies gilt im Besonderen für die Methanisierungsbakterien.

Bei der Rezirkulierung von Gärrest zur Anmaischung des Substrates kommt es in bestehenden Biogasanlagen häufig zu einer Aufkonzentration der Mineralien im Fermenter. Diese Mineralien haben teilweise die Eigenschaft von Hemmstoffen in Bezug auf den Biogasprozess.

Ein sehr problematischer Hemmstoff ist Ammoniak. Die Rezirkulierung von Gärrest ist besonders beim Einsatz von Substraten mit hohen Stickstoffanteilen kritisch. Beim Biogasprozess wird der organisch gebundene Stickstoff in Ammonium umgewandelt, der wiederum mit Ammoniak im chemischen Gleichgewicht steht. Dieses Gleichgewicht ist im Wesentlichen von der Temperatur und dem pH-Wert abhängig. Das Ammoniak ist für die Bakterien giftig und Ursache der so genannten Stickstoffhemmung. Das durch die Rezirkulierung aufkonzentrierte Ammonium und die vermehrte Ammoniakbildung behindern den Biogaserzeugungsprozess und können im Extremfall zu einem Abbruch der Biogasbildung führen. Des Weiteren kommt es durch die hohen Ammoniumkonzentrationen im Lager des flüssigen Gärrestes zu einer ständigen Ammoniakbildung und diese führt zu Ammoniakemissionen.

Großteilige Fremdstoffe werden in der Regel vor dem Eintrag in die Biogasanlage abgeschieden. Hingegen gelangen kleinteilige Fremdstoffe mit dem Substrat in die Fermenter und führen dort zu Ablagerungen. Speziell die Ablagerung von Schlämmen und Sand stellt ein großes Problem dar. Ursache für die Ablagerungen von Fremdstoffen im Fermenter ist zum einen die Größe der Fremdstoffe, die eine Abtrennung durch Siebe oder Rechen nicht ermöglichen. Weiterhin sind sie in der Regel unmagnetisch und elektrisch neutral. Eine Abscheidung durch Schwerkraft ist oftmals nicht möglich, da sie an Biomasse gebunden sind und somit der Dichteunterschied zu Wasser bzw. zur angemaischten Biomasse zu gering ist. Weiterhin sind die Ablagerungen im Fermenter auch problematisch, da die vorhandenen automatischen Entnahmesysteme wie Schieber oder Kratzböden nur unzureichend funktionieren. Ursache hierfür ist, dass die Ablagerungen durch die Schieber aufgewirbelt werden und somit zum Großteil im Fermenter verbleiben. Die Anreicherung der Ablagerungen führt dann zu einer Überlastung der Entnahmesysteme bis hin zum Ausfall und es verbleibt nur die manuelle Entnahme der Ablagerungen, wozu der Betrieb der Blogasanlage eingestellt werden muss.

Es sind Anstrengungen unternommen worden, die Situation zu verbessern und den Prozess der Erzeugung von Biogas aus organischen Abfallstoffen zu intensivieren. Die WO 03/042117 beschreibt ein integriertes anaerobes Faulsystem, bei dem cellulosehaltiges Ausgangsmaterial anaerob und unter Druck von Bakterien in Methan und Flüssigprodukt umgewandelt wird. Verschiedene Varianten werden in den Ansprüchen 1, 34, 51, 67 dargestellt und in 4 Abbildungen erläutert. Nicht gelöst in der Erfindung sind u.a. die vollständige Fremdstoffabtrennung aus festen Inputstoffen, die Gefahr des Ausschwemmens des biologisch aktiven Materials sowie die Hygienisierung des gesamten Gärrestes.

In der DE 195 47 320 wird mit dem Ziel, keimfreien und ammoniakfreien Flüssigdünger aus biologischen Rest- und Abfallstoffen zu erzeugen, in einem achtstufigen Fermenter ausgegorene biologische Material vier Stunden gekocht wird, um den Ammoniak auszutreiben. Die Vergärung erfolgt durch Bakterien, die auf einem Träger immobilisiert sind. Der ganze Prozess ist technisch ungewöhnlich aufwändig, insbesondere aus energetischer Sicht und erscheint kaum praktikabel.

Das Ziel der Erfindung besteht darin, die bestehenden Nachteile des Biogaserzeugungsprozesses zu überwinden und Substrate mit hohen Feststoff- und Stickstoffanteilen in einer Biogasanlage energetisch vorteilhaft zu nutzen. Dabei soll gewährleistet werden, dass folgende Punkte umgesetzt werden:
■ Steigerung der Biogasproduktion durch
   o Abtrennung von Störstoffen,
   o Optimierung der Biologie,
   o Reduzierung der Hemmstoffe (im Besonderen die Vermeidung der Stickstoffanreicherung in Biogasanlagen);
■ wesentliche Wassereinsparung;
■ vorteilhaftes Energieregime;
■ Vermeidung von Ammoniakemissionen;
■ Verringerung des Gärresteanfalls.

Die Erfindung hat die Aufgabe, den Biogasprozess im regulären Betrieb im Wesentlichen ohne ständigen Zusatz von Flüssigkeit abzusichern. Damit kann die Gärrestemenge reduziert werden. Der Gärrest soll am Ende des Verfahrens hygienisiert sein und sich durch nur geringe Emissionen (wie z. B. Ammoniak) auszeichnen.

Weiterhin soll Biomasse mit hohen Fremdstoffanteilen nutzbar sein.

Die Erfindung soll weiterhin die Aufgabe lösen, Substrate mit hohen Feststoffanteilen zur Biogasproduktion zu nutzen. Ferner soll der Einsatz stickstoffhaltiger Substrate (siehe Tabelle 1) möglich sein.

Der Energiehaushalt des gesamten Prozesses soll so gestaltet werden, dass bei nur geringen Verlusten an Wärme ein technisch, ökonomisch und ökologisch akzeptables Ergebnis erzielt wird.

Die Aufgabe wird erfindungsgemäß nach den Hauptansprüchen gelöst. Die Zeichnungen dienen dem Verständnis der Erfindung.

Das erfindungsgemäße Verfahren läuft im Einzelnen wie folgt ab:

Die Biomasse (Substrate) wird als Feststoff in den Prozessablauf eingebracht (1, 2). Um einen optimalen Biogasprozess zu ermöglichen, werden die Feststoffe zerkleinert (3). Mit Ausnahme von Anfahrvorgängen wird der Erfindung keine Flüssigkeit zugeführt. Der Biogasprozess läuft in einem pumpfähigen/flüssigen Zustand der Substrate ab. In einem Mischer (4) werden die zerkleinerten Substrate mit Rezirkulat angemaischt. Nach dem Mischer werden die Substrate nochmals durch einen Inline-Zerkleinerer (5) in ihrer Größe reduziert, um die aktive Oberfläche zu vergrößern und mit einer Pumpe (6) weiter befördert.

Das Rezirkulat ist nur in der Anfahrphase des erfindungsgemäßen Prozesses durch Wasser oder Gülle ersetzt.

In der Vorstufe, die als Zyklon (7) ausgeführt ist, erfolgen die Hydrolyse und die Säurebildung sowie die Abtrennung von Störstoffen. Die Hydrolyse und die Säurebildung sind die ersten Abbaustufen im Biogasprozess. Durch den Beginn des biologischen Abbaus kommt es zu einer Trennung von organischen und anorganischen Bestandteilen des Substrates. Die schwereren anorganischen Bestandteile, z. B. Sand, werden durch den Zyklon abgetrennt und stören den weiteren Biogasprozess nicht mehr. Die Verweilzeit des Substrates in der Vorstufe beträgt bis zu 6 Tage. Gleichzeitig wird eine Optimierung des Biogasprozesses durch die räumliche Trennung der verschiedenen Abbaustufen erreicht, wodurch sich für die spezifischen Bakterien jeweils die optimalen Milieubedingungen einstellen lassen. In den nachfolgenden Fermentern (8, 9) laufen die Essigsäurebildung und Methanogenese, d. h. die Bildung des Biogases, ab. In den Fermentern wird das Substrat mittels Rührwerken umgewälzt. Die Vergärung der Substrate erfolgt mesophil, d. h. bei Temperaturen von 38 bis 42 °C, oder thermophil, d. h. bei Temperaturen von 50 bis 60 °C. Die Verweilzeit in den Fermentern beträgt zwischen 20 und 40 Tagen. Anzahl und Größe der Fermenter sowie die Verweilzeit der Substratmischung sind abhängig von Art und Menge der Substrate.

Das Biogas wird in Gasblasen (9) gespeichert und nach einer Reinigung (19) in einer Kraftwärmekopplungsanlage (20) in Strom und Wärme umgewandelt. In der Regel werden Blockheizkraftwerke eingesetzt, doch können auch Mikrogasturbinen, Gasturbinen, Brennstoffzellen oder ORC-Prozesse zum Einsatz kommen.

Der Gärrest des Biogasprozesses wird zum Anmaischen der Substrate im Mischer (4) verwendet. Hierbei wird der Gärrest in zwei Teilströme getrennt.

Teilstrom 1 (A) ist unbehandelter Gärrest. Durch diesen wird das Neusubstrat biologisch angeimpft, was einen reibungslosen Ablauf des Biogasprozesses unterstützt. Das hat den großen Vorteil, dass Bakterien in die Anmaischvorrichtung kommen, die dem bislang verwendeten Substrat besonders gut entsprechen, dieses zügig abbauen und eine hohe Biogasproduktion fördern.

Teilstrom 2 (B) wird erst einer Substrataufbereitung (10) unterzogen, um die Hemmstoffe zu entfernen. Hierbei geht es in erster Linie um die Entfernung von Ammonium/ Ammoniak mit Hilfe eines Strippprozesses. Der Strippprozess wird mit der Abwärme der KWK-Anlage (20) beheizt. Gleichzeitig wird eine Hygienisierung des Gärrestes erreicht, d. h. die Temperatur beim Strippprozess liegt für einen Zeitraum von mehr als 60 Minuten über 70 °C. Daher arbeitet der Strippprozess in einem Batchbetrieb. Der Abscheidegrad des Ammoniumstickstoffs soll bei ca. 50 bis 90 % liegen. Um die Heizenergie für den Strippprozess zu minimieren, werden der zu- und abgeführte Gärrest im Gegenstromverfahren vorgewärmt bzw. abgekühlt (11). Um das Gegenstromverfahren zu ermöglichen, sind für den Strippprozess 3 Strippbehälter vorgesehen, von denen maximal 2 Behälter in Betrieb sind bzw. 1 Behälter in Betrieb und jeweils 1 Behälter befüllt und entleert wird. Der hygienisierte und stickstoffarme Gärrest wird über einen Pufferbehälter (12) kontinuierlich einem Separator (13) zugeführt. Alternativ kann der Strippprozess mit vier Strippbehältern ausgeführt werden, wobei jeweils 1 Behälter befüllt und entleert wird sowie 2 Behälter in Betrieb sind. Bei dieser Variante entfällt der Pufferbehälter (12) vor dem Separator (13).

Durch den Separator (13) wird der Gärrest in eine Fest- und Flüssigphase getrennt. Der Trockensubstanzanteil der Flüssigphase beträgt etwa 2 bis 6 %. Sie wird zum Anmaischen des Substrates verwendet. Durch den Strippprozess wird gleichzeitig eine thermische Desintegration der Biomasse des Gärrestes erreicht. Hierdurch werden eine weitere Umwandlung der rezirkulierten Biomasse der Flüssigphase im Fermenter erreicht und somit erhöhte Gaserträge erzielt.

Der Trockensubstanzanteil der Festphase beträgt etwa 25 bis 50 %. Durch den Strippprozess ist die Ammoniumkonzentration in der Festphase auf ca. 0,5 bis 10 kg NH₄ / t TS reduziert. Damit sind die Ammoniakemissionen deutlich vermindert. Zum Vergleich liegen die Ammoniumkonzentrationen bei unbehandeltem flüssigem Gärrest bei ca. 20 bis 80 kg NH₄ / t TS. Die Geruchsemissionen werden durch den Abbau der Substrate im Biogasprozess und die Hygienisierung reduziert. Ein weiterer aerober Abbau des Gärrestes und somit die Entstehung von Geruchsemissionen wird durch Lufttrocknung des Gärrestes mit der Abwärme (14) aus dem Strippprozess verhindert. Der feste Gärrest wird gelagert und kann in der Landwirtschaft als Dünger ausgebracht werden.

Der Biogasprozess muss beheizt werden. Aufgrund der Massenverhältnisse zwischen Substrat und Rezirkulat erfolgt der wesentliche Wärmeeintrag in den Biogasprozess durch den Strippprozess. Weiterhin wird die Abwärme des Strippprozesses zur Beheizung des Biogasprozesses verwendet. Alternativ kann auch direkt die Wärme der Kraftwärmekopplungsanlage (20) zur Beheizung verwendet werden. Die Beheizung erfolgt durch einen externen Wärmeübertrager (18). Hierzu wird Substrat aus der Vorstufe und den Fermentern entnommen, im Wärmeübertrager aufgeheizt und zurück gepumpt. Die Behälter werden abwechselnd beheizt und das Substrat dem Behälter wieder zugeführt, dem es entnommen wurde. Beim Zyklon wird das Substrat zur Beheizung am Ausgang entnommen, um die Schmutzfracht und somit auch die Verschmutzung des Wärmetauschers möglichst gering zu halten.

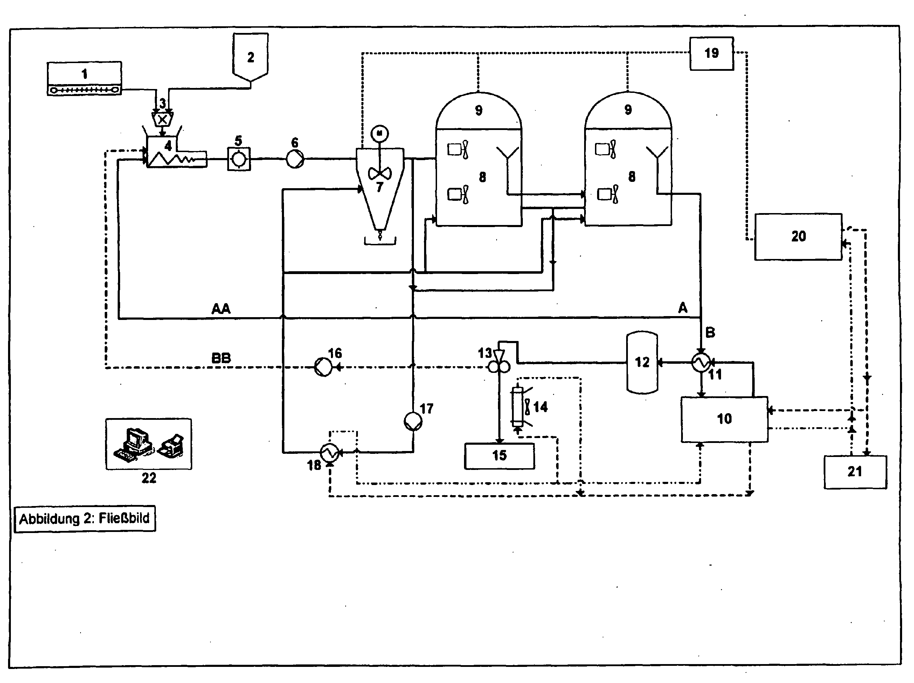

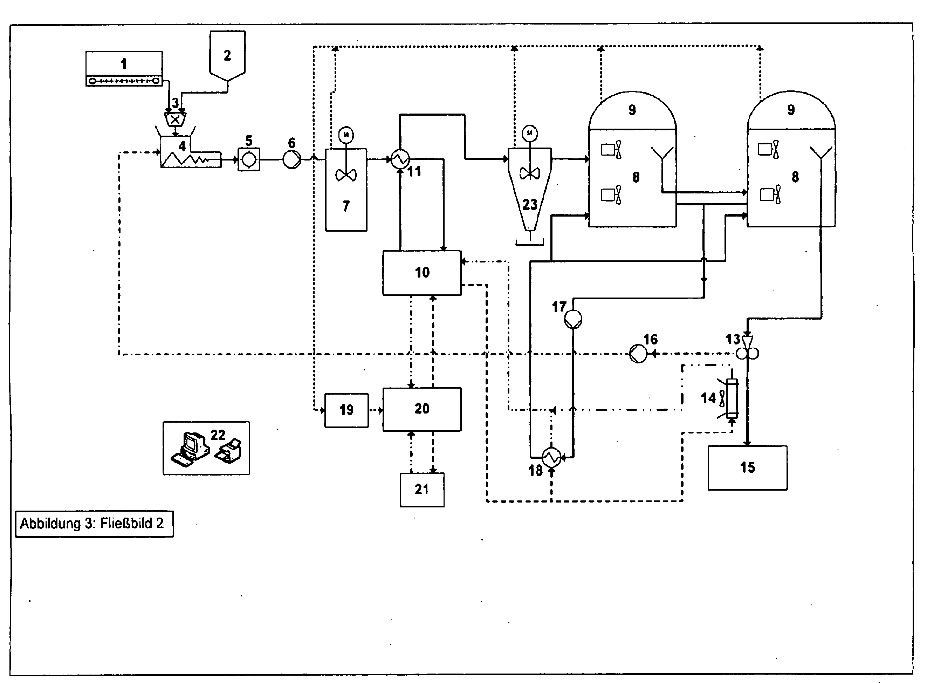

Als Variante zu der Schaltung in Abbildung 2 wird das Verfahren entsprechend der Abbildung 3 gestaltet werden. Bei dieser Variante wird die biologische Vorstufe (7) und die Sandabscheidung (23) apparatetechnisch getrennt. Nach der biologischen Vorstufe (7) wird das Substrat einer Substrataufbereitung (10) unterzogen. Die Substrataufbereitung ist wie in der zuvor beschriebenen Variante ausgestaltet.

Das hygienisierte und stickstoffarme Substrat wird dem Störstoffabscheider (23) zugeführt. Alternativ zur Abbildung 3 kann der Störstoffabscheider (23) auch zwischen der Substrataufbereitung (10) und dem Gegenstromwärmeübertrager (11) angeordnet werden. Vom Störstoffabscheider wird das Substrat, welches hygienisiert und von Hemm- und Störstoffen befreit ist, den nachfolgenden Fermentern (8, 9) zur Biogasbildung zugeführt. Die Vergärung erfolgt wie zuvor beschrieben. Der Gärrest des Biogasprozesses (Ablauf des Fermenters) wird dem Separator (13) zur Trennung des Gärrestes in eine Fest- und Flüssigphase zugeführt. Die Flüssigphase wird zum Anmaischen des Substrates verwendet. Auf eine Rezirkulierung in 2 Teilströmen kann bei dieser Variante verzichtet werden. Der Rest des Verfahrens in Abbildung 3 bleibt im Vergleich zum Verfahren in Abbildung 2 unverändert.

Als weitere Variante ergibt sich die Entfernung der Störstoffe sowohl nach der Biogasgewinnung (Abbildung 2) als auch nach der Hydrolyse (Abbildung 3), um einen besonders hohen Abscheidegrad bezüglich der Hemmstoffe zu erzielen.

### Ausführungsbeispiel

Das Beispiel für die Ausführung des Verfahrens verwendet Hühnermist als Substrat, doch ist es ebenfalls zur Demonstration für den Einsatz aller anderen bereits genannten Substrate mit hohen Stickstoff- oder hohen Feststoffanteilen geeignet (siehe Abbildung Bilanz).

Der Einsatz von Hühnermist ist dadurch gekennzeichnet dass dieses Substrat einen hohen Trockensubstanzanteil (TS = 30 % bis 80 %) und einen hohen Stickstoffanteil von bis zu 6 % TS hat. In der vorliegenden Beispielrechnung beträgt der Trockensubstanzanteil 48 % bei einem Stickstoffanteil von 5 % TS.

In Abwandlung des beschriebenen Verfahrens wird der Hühnermist ohne Zerkleinerung und Mischer direkt der Vorstufe zugeführt. In der Vorstufe wird ein pumpfähiges Substrat mit einem Trockensubstanzanteil von TS = 10 bis 15 % durch Anmaischen mit Rezirkulat erzeugt. In der Beispielrechnung beträgt der Trockensubstanzanteil 12,5 %, wobei zum Anfahren der Anlage ca. 2.000 t Rezirkulat bzw. beim erstmaligen Anfahren Wasser benötigt werden. Die Durchmischung erfolgt durch das Zentralrührwerk des Zyklons.

Die Verweilzeit des Substrates im Zyklon beträgt im Anwendungsbeispiel 4 Tage. Im Zyklon setzt sich der im Hühnermist gebundene Sand ab. Es werden laut Beispiel jährlich ca. 860 t Sand aus dem Prozess abgetrennt.

Im nachfolgenden Inline-Zerkleinerer erfolgt die Einstellung der erforderlichen Partikelgröße des Substrates. Zerkleinert werden im Inline-Zerkleinerer im Wesentlichen Stroh und Federn, die soweit zerkleinert werden, dass durch die Partikelgröße eine gute Pumpfähigkeit und ein störungsfreier Substratfluss in den Rohrleitungen sichergestellt sind. Eine dem Inline-Zerkleinerer nachgeschaltet Pumpe fördert das Substrat in den Fermenter.

Die Verweilzeit des Substrates in den Fermentern beträgt im Anwendungsfall 30 Tage. Der Prozess verläuft mesophil bei 40 °C. Die Ammoniumkonzentration im Fermenter beträgt ca. 4 kg NH₄-N / t Substrat.

Das erzeugte Biogas wird, bevor es in einem Blockheizkraftwerk verwertet wird, in einer externen Gasreinigung aufbereitet. Um beim Ausfall der Wärme-Kraftmaschine den Verbrauch des Biogases und die Beheizung der Substrataufbereitung sicher zu stellen, sind eine Notfackel und eine Notheizung installiert. Der elektrische Strom wird ins Netz eingespeist, während die Wärme zur Versorgung der Substrataufbereitung und weiterer Verbraucher dient.

Nach der Fermentation wird der Teilstrom 1 des vergorenen Substrates mit einem Trockensubstanzanteil von 7,5 % rezirkuliert und dem Mischer wieder zugeführt. Dadurch werden die für die Fermentation notwendigen Bakterien dem Neusubstrat beigemischt. Das restliche Substrat (Teilstrom 2) wird der Substrataufbereitung zugeführt. Dieses wird dort hygienisiert und jährlich ca. 310 t Stickstoff entfernt. Danach folgt die Separation in Flüssig- und Festphase. Die rezirkulierte Flüssigphase weist noch eine Ammoniumkonzentration von ca. 0,8 kg NH₄-N / t Substrat bei einem Trockensubstanzanteil von 3 % auf. Sie wird ebenso wie Teilstrom 1 zum Anmaischen der Neusubstrate verwendet.

Durch die Mischung des Neusubstrates mit dem Rezirkulat von Teilstrom 1 und Teilstrom 2 im Mischer ergibt sich eine Temperatur des Gemisches von ca. 36 °C. Mit der Abwärme der Substrataufbereitung werden über einen Wärmeübertrager die Vorstufe und die Fermenter beheizt. Hierbei müssen durch die Beheizung die Erwärmung von 36 auf 40 °C sichergestellt und die Wärmeverluste der Biogasanlage ausgeglichen werden.

Die hygienisierte Festphase des Gärrestes hat im Anwendungsfall einen Trockensubstanzanteil von ca. 36 %. Sie wird nach einer Lufttrocknung in einem Feststofflager mit einem Trockensubstanzanteil von ca. 66 % gelagert und kann der Verwendung, z. B. in der Landwirtschaft, zugeführt werden. Als Wärme für die Lufttrocknung wird Abwärme aus dem Strippprozess verwendet.

Die Erfindung wird durch folgende Abbildungen näher erläutert:

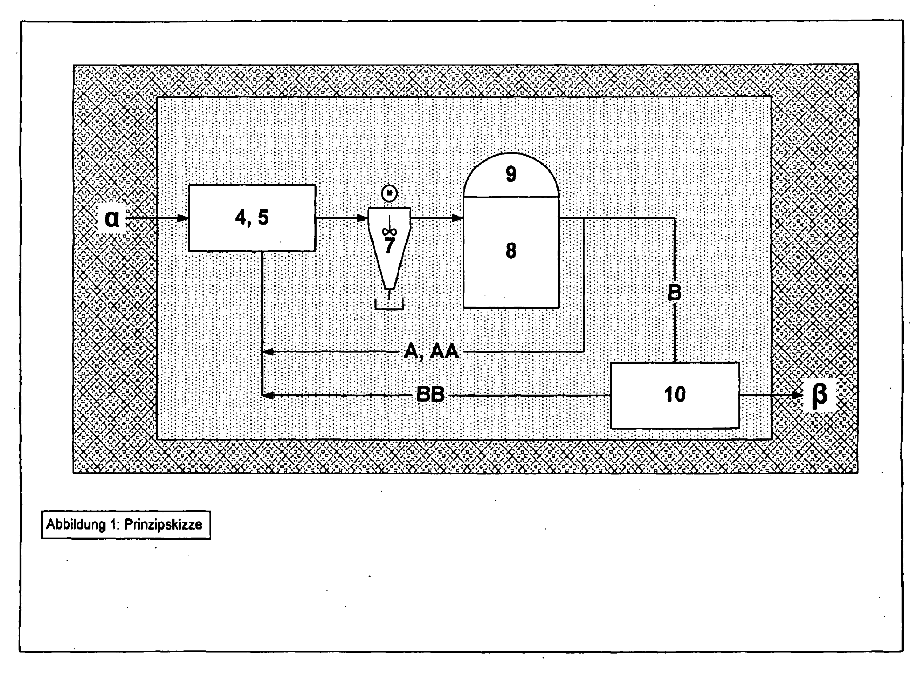

- Abbildung 1:: Prinzipskizze
- Abbildung 2:: Fließbild 1
- Abbildung 3:: Fließbild 2

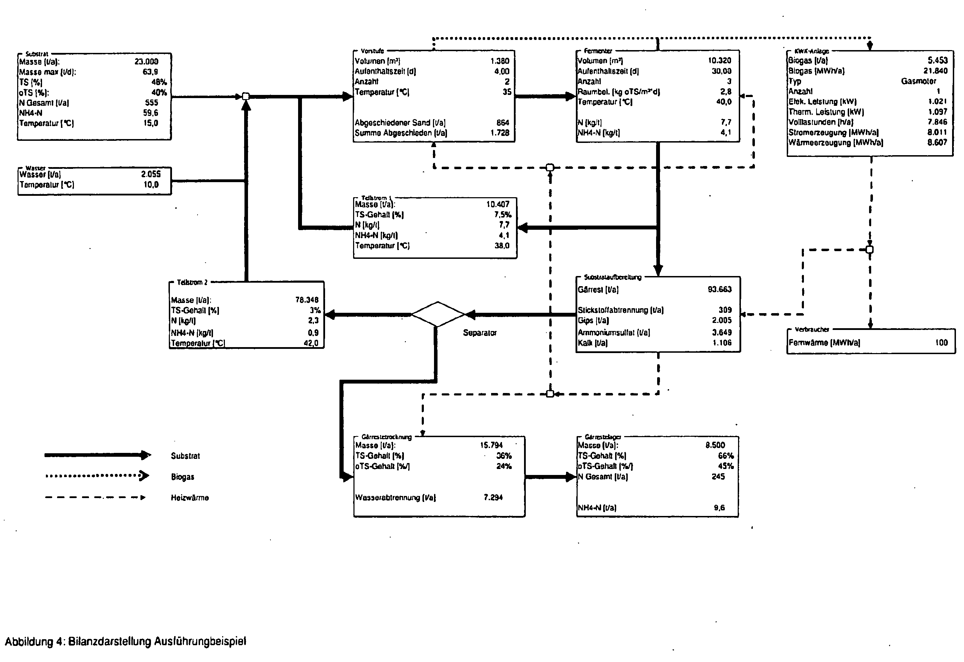

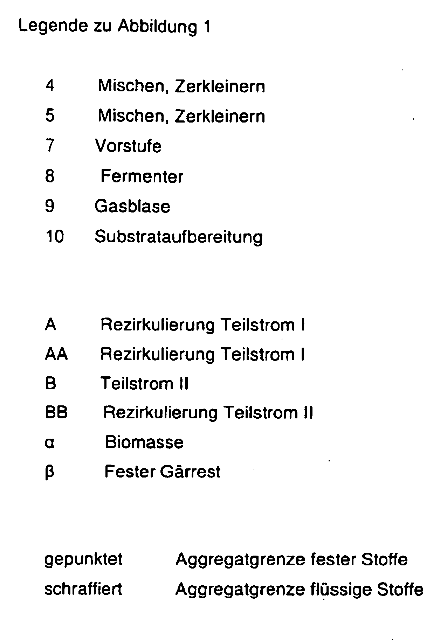

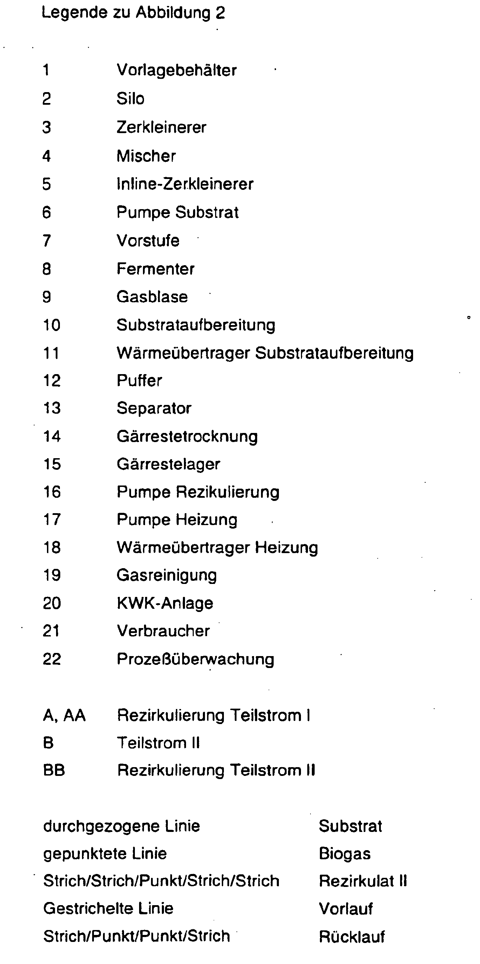

- Abbildung 4:: Bilanzdarstellung für das Ausführungsbeispiel

Die Erfindung weist gegenüber dem Stand der Technik wesentliche Vorzüge auf. Es ist nunmehr möglich, Substrate erfindungsgemäß zu nutzen, die in herkömmlichen Biogasverfahren nicht oder nur mit großen Nachteilen eingesetzt werden konnten.

Der ökonomische Vorteil der Erfindung liegt u. a. im geringeren Verbrauch von Wasser im Vergleich zu gängigen Nassvergärungsverfahren bei gleichzeitig verbesserter Biogaserzeugung und damit deutlich erhöhten Gaserträgen im Vergleich zu Trockenvergärungsverfahren.

Tabelle 2 stellt die Erfindung einer normalen Nassvergärung gegenüber. Wie aus der Tabelle ersichtlich, ergeben sich beispielhaft durch die Erfindung verringerte Investitionen für die Gärrestelagerung von ca. 480.000 €. Weiterhin verringern sich die jährlichen Kosten für das Wasser und die Ausbringung der Gärreste auf landwirtschaftliche Nutzflächen um ca. 458.000 EURO.

**Tabelle 2: Vergleich der Erfindung zu einer herkömmlichen Nassvergärung**

| | **Erfindung** | | **Nassvergärung** | |
|---|---|---|---|---|
| Hühnermist | 23.000 t/a | | 23.000 t/a | |
| Wasserverbrauch | 2.000 t/a | 4.000 €/a | 51.000 €/a | 102.000 €/a |
| Gärrestemenge | 8.500 t/a | | 68.500 t/a | |
| Lagerraum | 8.000 m³ | 170.000 € | 34.250 m³ | 650.000 € |
| Ausbringkosten für den Gärrest | 6 €/t | 51.000 €/a | 6 €/t | 411.000 €/a |

## Patentansprüche

1. Verfahren zur Nutzung von Biomasse in einem Biogasprozess,
indem zunächst als Feststoff vorliegende Biomasse in einem Zerkleinerer zerkleinert, angemaischt, mit Bakterien geimpft und entstandenes Biogas abgezogen wird, wobei
zerkleinerte Biomasse mit Rezirkulat in einem Mischer angemaischt und das Substrat in einen pumpfähigen Zustand überführt wird,
anschließend die pumpfähige Mischung in einem Inline-Zerkleinerer weiter homogenisiert wird,
in einem oder mehreren nachgeschalteten Abscheidern mit Rührwerk Hydrolyse und Säurebildung durch bakterielle Einwirkung ablaufen,
anorganische Bestandteile separiert und verworfen werden,
in Fermentern unter fortlaufender Durchmischung bei erhöhten Temperaturen Essigsäurebildung und Methanogenese ablaufen, wobei Biogas entsteht,
das Biogas abgezogen, ggfs. gespeichert und einer energetischen Nutzung zugeführt wird,
während entstandener Gärrest in zwei Teilströme geteilt wird, wobei Teilstrom 1 unbehandelt zum Anmaischen neuer Biomasse dient,
hingegen Teilstrom 2 einer Substrataufbereitung unterzogen wird,
indem ihm in einem Stripprozess Ammoniak/Ammoniumstickstoff entzogen wird,
der Teilstrom 2 nach dem Strippprozess ggfs. mittels Wärmeübertrager erwärmt und nunmehr als hygienisierter und stickstoffarmer Gärrest über einen Pufferbehälter kontinuierlich einem Separator zugeführt und in eine feste und flüssige Phase getrennt wird,
die flüssige Phase als Rezirkulat dem Mischer zugeführt wird,
die feste Phase entweder getrocknet oder als Suspension verwertet wird.
**dadurch gekennzeichnet, dass**
entsprechend Abbildung 2
zerkleinerte Biomasse mit Rezirkulat in einem Mischer (4) angemaischt und das Substrat in einen pumpfähigen Zustand übergeführt wird,
anschließend die pumpfähige Mischung in einem Inline-Zerkleinerer (5) weiter homogenisiert wird,
in einem oder mehreren nacheinander geschalteten Zyklonen mit Zentralrührwerk (7) Hydrolyse und Säurebildung durch bakterielle Einwirkung ablaufen,
anorganische Bestandteile separiert und verworfen werden,
in Fermentern (8) unter fortlaufender mechanischer Durchmischung bei einer Temperatur von ca. 40 °C Essigsäurebildung und Methanogenese ablaufen, wobei Biogas entsteht,
das Biogas abgezogen, gespeichert (9) und einer energetischen Nutzung in einer Kraft-Wärme-Kopplungsanlage KWK (20) zugeführt wird,
während entstandener Gärrest in zwei Teilströme getrennt wird,
wobei Teilstrom 1 (A, AA), der eine Temperatur von ca. 40 °C aufweist, unbehandelt zum Anmaischen neuer Biomasse dient,
hingegen Teilstrom 2 (B) einer Substrataufbereitung (10) unterzogen wird,
indem er auf ca. 70 bis 90 °C aufgeheizt wird,
ihm in einem Strippprozeß Ammoniak/Ammoniumstickstoff entzogen wird,
wobei die notwendige Wärme für den Strippprozeß aus der KWK-Anlage (20) kommt, der Strippprozeß selbst bei ca. 75 bis 90 °C abläuft,
der zu- und abgeführte Gärrest im Gegenstromverfahren (11) abgekühlt bzw. vorgeheizt wird,
der Teilstrom 2 nach dem Strippprozeß über den Wärmetauscher (11) mit einer Temperatur von ca. 45 °C als nunmehr hygienisierter und stickstoffarmer Gärrest über einen Pufferbehälter (12) kontinuierlich einem Separator (13) zugeführt und in eine feste und eine flüssige Phase getrennt wird,
die flüssige Phase als Rezirkulat (BB) dem Mischer (4) zugeführt wird,
die feste Phase entweder getrocknet (14) oder direkt als Suspension als Dünger in der Landwirtschaft verwendet wird.

2. Verfahren zur Nutzung von Biomasse in einem Biogasprozess
indem zunächst als Feststoff vorliegende Biomasse in einem Zerkleinerer zerkleinert, angemaischt, mit Bakterien geimpft und entstandenes Biogas abgezogen wird, wobei
zerkleinerte Biomasse mit Rezirkulat in einem Mischer angemaischt und das Substrat in einen pumpfähigen Zustand überführt wird,
anschließend die pumpfähige Mischung in einem Inline-Zerkleinerer weiter homogenisiert wird,
in einem oder mehreren nachgeschalteten Abscheidern mit Rührwerk Hydrolyse und Säurebildung durch bakterielle Einwirkung ablaufen,
anorganische Bestandteile separiert und verworfen werden,
in Fermentern unter fortlaufender Durchmischung bei erhöhten Temperaturen Essigsäurebildung und Methanogenese ablaufen, wobei Biogas entsteht,
das Biogas abgezogen, ggfs. gespeichert und einer energetischen Nutzung zugeführt wird,
während entstandener Gärrest in zwei Teilströme geteilt wird, wobei Teilstrom 1 unbehandelt zum Anmaischen neuer Biomasse dient,
hingegen Teilstrom 2 einer Substrataufbereitung unterzogen wird,
indem ihm in einem Stripprozess Ammoniak/Ammoniumstickstoff entzogen wird,
der Teilstrom 2 nach dem Strippprozess ggfs. mittels Wärmeübertrager erwärmt und nunmehr als hygienisierter und stickstoffarmer Gärrest über einen Pufferbehälter kontinuierlich einem Separator zugeführt und in eine feste und flüssige Phase getrennt wird,
die flüssige Phase als Rezirkulat dem Mischer zugeführt wird,
die feste Phase entweder getrocknet oder als Suspension verwertet wird,
**dadurch gekennzeichnet, dass** entsprechend Abbildung 3
zerkleinerte Biomasse mit Rezirkulat in einem Mischer (4) angemaischt und das Substrat in einen pumpfähigen Zustand überführt wird,
anschließend die pumpfähige Mischung in einem Inline-Zerkleinerer (5) weiter homogenisiert wird,
in einer oder mehrerer nacheinander geschalteter Vorstufen (7) mit Zentralrührwerk Hydrolyse und Säurebildung durch bakterielle Einwirkung ablaufen,
die entstandene Suspension anschließend einer Substrataufbereitung (10) unterzogen wird,
indem sie auf 70 bis 90 °C aufgeheizt wird,
ihr in einem Strippprozess Ammoniak/Ammoniumstickstoff entzogen wird,
wobei die notwendige Wärme für den Strippprozess aus der KWK-Anlage (20) kommen kann,
der Strippprozess bei 70 bis 90 °C abläuft,
der zu- und abgeführte Gärrest im Gegenstromverfahren (11) abgekühlt bzw. vorgewärmt wird,
in einem Abscheider (23) anorganische Bestandteile separiert und verworfen werden, in Fermentern (8) unter fortlaufender Durchmischung bei einer Temperatur von 30 bis 60 °C Essigsäurebildung und Methanogenese ablaufen,
das Biogas abgezogen, gespeichert (9) und einer energetischen Nutzung zugeführt wird,
der nunmehr hygienisierte und stickstoffarme Gärrest kontinuierlich einem Separator (13) zugeführt und in eine feste und flüssige Phase getrennt wird,
die flüssige Phase als Rezirkulat dem Mischer (4) zugeführt wird,
die feste Phase entweder getrocknet (14) oder direkt als Suspension verwendet wird.

3. Verfahren nach einem oder mehreren der Ansprüche 1 bis 2,
**dadurch gekennzeichnet, dass** beim Anfahren des kontinuierlichen Biogasprozesses anstelle des Gärrestes Wasser oder Gülle zum Anmaischen verwendet wird.

4. Verfahren einem oder mehreren der Ansprüche 1 bis 3,
**dadurch gekennzeichnet, dass** die Verweilzeit in der Vorstufe ein bis sechs Tage beträgt.

5. Verfahren nach einem oder mehreren der Ansprüche 3 bis 4
**dadurch gekennzeichnet, dass** mit dem Teilstrom 1 das Substrat im Mischer (4) oder im Fermenter (8) bakteriell geimpft wird.

6. Verfahren nach einem oder mehreren der Ansprüche 1 bis 5,
**dadurch gekennzeichnet, dass** die Vergärung im Fermenter (8) mesophil bei Temperaturen zwischen 35 und 42 °C oder thermophil bei Temperaturen zwischen 50 und 60 °C erfolgt.

7. Verfahren nach einem oder mehreren der Ansprüche 1 bis 6,
**dadurch gekennzeichnet, dass** in mehreren Fermentern (8) unabhängig voneinander mesophil und thermophil gearbeitet wird und die Verweilzeit in den Fermentern (8) 20 bis 40 Tage beträgt.

8. Verfahren nach einem oder mehreren der Ansprüche 1 bis 7,
**dadurch gekennzeichnet, dass** der Strippprozeß bei einer Temperatur > 70 °C und einer Zeitdauer > 1 Stunde als Batchprozeß abläuft.

9. Verfahren nach einem oder mehreren der Ansprüche 1 bis 8,
**dadurch gekennzeichnet, dass** der Strippprozeß bezüglich der Gärrestezu- und -abführung im thermischen Gegenstromverfahren abläuft.

10. Verfahren nach einem oder mehreren der Ansprüche 1 bis 9,
**dadurch gekennzeichnet, dass** von drei Strippbehältern jeweils einer befüllt und betrieben und entleert ist oder einer leer ist und zwei betrieben werden, von vier Strippbehältern jeweils einer befüllt und entleert wird und zwei betrieben werden und der Pufferbehälter entfällt.

11. Verfahren nach einem oder mehreren der Ansprüche 1 bis 10,
**dadurch gekennzeichnet, dass** mit Restwärme vom Strippprozeß die Vorstufe (7) und die Fermenter (8) beheizt und die feste Phase im Gärrestelager (15) getrocknet werden.

12. Verfahren nach einem oder mehreren der Ansprüche 1 bis 11,
**dadurch gekennzeichnet, dass** als Biomasse mit hohem Fremdstoffanteil Geflügelmist eingesetzt wird, dieser direkt der Vorstufe (7) zugeführt wird, dort die Durchmischung erfolgt und die Mischung anschließend dem Inline-Zerkleinerer zugeführt wird.

## Claims

1. Method for using biomass in a biogas process,
by biomass present as solid first being comminuted in a comminuter, mashed, inoculated with bacteria and the resultant biogas being taken off,
wherein
comminuted biomass is mashed with recirculated material in a mixer and the substrate is converted into a pumpable state,
then the pumpable mixture is further homogenized in an inline comminuter,
hydrolysis and acid formation proceed by bacterial action in one or more downstream separators equipped with an agitator,
inorganic components are separated and discarded, acetic acid formation and methanogenesis proceed in fermenters with continuing mixing at elevated temperatures, wherein biogas is formed,
the biogas is taken off, optionally stored and fed to a use as energy,
while the resultant fermentation residue is divided into two substreams, wherein substream 1 serves untreated for mashing new biomass,
in contrast substream 2 is subjected to substrate preparation,
by removing therefrom ammonia/ammonium nitrogen in a stripping process,
substream 2, after the stripping process, is optionally warmed by means of a heat exchanger and then continuously fed as a sanitized and low-nitrogen fermentation residue via a buffer tank to a separator and separated into a solid phase and a liquid phase, the liquid phase is fed as recirculated material to the mixer,
the solid phase is either dried or utilized as suspension,
**characterized in that**,
corresponding to figure 2,
comminuted biomass is mashed with recirculated material in a mixer (4) and the substrate is converted into a pumpable state,
the pumpable mixture is then further homogenized in an inline comminutor (5),
hydrolysis and acid formation proceed by bacterial action in one or more series-connected cyclones having a central agitator (7),
inorganic components are separated and discarded, acetic acid formation and methanogenesis proceed in fermenters (8) with continuous mechanical mixing at a temperature of approximately 40°C, wherein biogas is formed,
the biogas is taken off, stored (9) and fed to use as energy in a combined heat and power (CHP) plant (20), whereas fermentation residue formed is separated into two substreams,
wherein substream 1 (A, AA) which has a temperature of approximately 40°C serves untreated for mashing new biomass,
in contrast, substream 2 (B) is subjected to a substrate preparation (10),
by heating it to approximately 70 to 90°C,
taking off ammonia/ammonium nitrogen therefrom in a stripping process,
wherein the necessary heat for the stripping process comes from the CHP plant (20), the stripping process itself proceeds at approximately 75 to 90°C,
the fermentation residue that is fed in and conducted out is cooled or preheated in a counterflow process (11),
substream 2 downstream of the stripping process is continuously fed via the heat exchanger (11) at a temperature of approximately 45°C as a now sanitized and low-nitrogen fermentation residue via a buffer tank (12) to a separator (13) and separated into a solid phase and a liquid phase,
the liquid phase is fed as recirculated material (BB) to the mixer (4),
the solid phase is either dried (14) or used directly as suspension as fertilizer in agriculture.

2. Method for using biomass in a biogas process,
by biomass present as solid first being comminuted in a comminuter, mashed, inoculated with bacteria and the resultant biogas being taken off,
wherein
comminuted biomass is mashed with recirculated material in a mixer and the substrate is converted into a pumpable state,
then the pumpable mixture is further homogenized in an inline comminuter,
hydrolysis and acid formation proceed by bacterial action in one or more downstream separators equipped with an agitator,
inorganic components are separated and discarded, acetic acid formation and methanogenesis proceed in fermenters with continuing mixing at elevated temperatures, wherein biogas is formed,
the biogas is taken off, optionally stored and fed to a use as energy,
while the resultant fermentation residue is divided into two substreams, wherein substream 1 serves untreated for mashing new biomass,
in contrast substream 2 is subjected to substrate preparation,
by removing therefrom ammonia/ammonium nitrogen in a stripping process,
substream 2, after the stripping process, is optionally warmed by means of a heat exchanger and then continuously fed as a sanitized and low-nitrogen fermentation residue via a buffer tank to a separator and separated into a solid phase and a liquid phase, the liquid phase is fed as recirculated material to the mixer,
the solid phase is either dried or utilized as suspension,
**characterized in that**, corresponding to Figure 3, comminuted biomass is mashed with recirculated material in a mixer (4) and the substrate is converted into a pumpable state,
the pumpable mixture is then further homogenized in an inline comminutor (5),
hydrolysis and acid formation proceed by bacterial action in one or more series-connected preliminary stages (7) having a central agitator,
the resultant suspension is then subjected to a substrate preparation (10),
by heating it to 70 to 90°C,
taking off ammonia/ammonium nitrogen therefrom in a stripping process,
wherein the necessary heat for the stripping process can come from the CHP plant (20),
the stripping process proceeds at 70 to 90°C,
the fermentation residue that is fed in and conducted away is cooled or preheated in a countercurrent process (11),
inorganic components are separated and discarded in a separator (23),
acetic acid formation and methanogenesis proceed at a temperature of 30 to 60°C in fermenters (8) with continuing mixing,
the biogas is taken off, stored (9) and fed to a use as energy,
the then sanitized and low-nitrogen fermentation residue is continuously fed to a separator (13) and separated into a solid phase and a liquid phase,
the liquid phase is fed as recirculated material to the mixer (4),
the solid phase is either dried (14) or used directly as suspension.

3. Method according to one or more of Claims 1 to 2, **characterized in that**, during startup of the continuous biogas process, water or liquid manure is used for the mashing instead of the fermentation residue.

4. Method according to one or more of Claims 1 to 3, **characterized in that** the residence time in the preliminary stage is one to six days.

5. Method according to one or more of Claims 3 to 4, **characterized in that** the substrate is bacterially inoculated with the substream 1 in the mixer (4) or the fermenter (8).

6. Method according to one or more of Claims 1 to 5, **characterized in that** the fermentation in the fermenter (8) proceeds mesophilically at temperatures between 35 and 42°C or thermophilically at temperatures between 50 and 60°C.

7. Method according to one or more of Claims 1 to 6, **characterized in that**, in a plurality of fermenters (8), independently of one another, mesophilic and thermophilic conditions are employed and the residence time in the fermenters (8) is 20 to 40 days.

8. Method according to one or more of Claims 1 to 7, **characterized in that** the stripping process proceeds as a batch process at a temperature > 70°C and for a time period > 1 hour.

9. Method according to one or more of Claims 1 to 8, **characterized in that** the stripping process proceeds in a thermal counterflow process with respect to the feeding in and conducting away of the fermentation residues.

10. Method according to one or more of Claims 1 to 9, **characterized in that**, of three stripping vessels, one in each case is filled and operated and emptied, or one is empty and two are being operated, of four stripping vessels, one in each case is being filled and emptied and two are being operated and the buffer vessel is omitted.

11. Method according to one or more of Claims 1 to 10, **characterized in that**, using residual heat from the stripping process, the preliminary stage (7) and the fermenter (8) are heated and the solid phase in the fermentation residues store (15) is dried.

12. Method according to one or more of Claims 1 to 11, **characterized in that**, as biomass having a high foreign substance fraction, poultry manure is used, this is fed directly to the preliminary stage (7), there the mixing proceeds and the mixture is then fed to the inline comminutor.

## Revendications

1. Procédé pour l'utilisation de la biomasse dans un procédé de production de biogaz,
dans lequel d'abord une biomasse, présente sous forme d'une matière solide, est broyée dans un broyeur, empâtée, on y inocule des bactéries, et on soutire le biogaz formé,
où
la biomasse broyée, avec le recirculat, est empâtée dans un mélangeur, et le substrat est converti pour prendre un état apte au pompage,
puis le mélange apte au pompage est soumis à une homogénéisation plus poussée dans un broyeur en ligne, on procède à une hydrolyse et à une formation d'acide sous l'action de bactéries dans un ou plusieurs séparateurs montés en aval et munis d'un agitateur,
les constituants inorganiques sont séparés et jetés,
on procède dans des fermenteurs, en présence d'un mélange continu à haute température, à la formation d'acide acétique et à une méthanogenèse, avec formation d'un biogaz,
le biogaz est soutiré, éventuellement stocké, et envoyé à une utilisation énergétique,
tandis que le résidu de fermentation qui se forme est subdivisé en deux courants partiels, le courant partiel 1 restant non traité pour empâtage de la nouvelle biomasse, tandis que le courant partiel 2 est soumis à une préparation de substrat, par le fait qu'on en soutire, dans une opération d'épuisement, de l'ammoniac/de l'azote ammoniacal,
le courant partiel 2 est, après l'opération d'épuisement, éventuellement chauffé à l'aide d'un caloporteur, et maintenant, sous forme d'un résidu de fermentation hygiénisé et à faible teneur en azote, est envoyé en continu dans un séparateur par l'intermédiaire d'un récipient tampon, et est séparé en une phase solide et une phase liquide,
la phase liquide est envoyée au mélange sous forme de recirculat,
la phase solide est séchée, ou valorisée sous forme d'une suspension,
**caractérisé en ce que**, d'une manière correspondant à la Figure 2, la biomasse broyée, avec le recirculat, est empâtée dans un mélangeur (4), et le substrat est converti pour prendre un état apte au pompage, puis le mélange apte au pompage est soumis à une homogénéisation plus poussée dans un broyeur en ligne (5),
dans un ou plusieurs cyclones montés en aval les uns des autres, avec agitateur central (7), on procède à une hydrolyse et à une formation d'acide sous l'action de bactéries,
les constituants inorganiques sont séparés et jetés,
dans des fermenteurs (8), en présence d'un mélange intime mécanique continu et à une température d'environ 40°C, on procède à la formation d'acide acétique et à une méthanogenèse, avec formation d'un biogaz,
le biogaz est soutiré, stocké (9) et envoyé à une utilisation énergétique dans une installation de cogénération d'électricité et d'énergie thermique KWK (20),
tandis que le résidu de fermentation qui se forme est subdivisé en deux courants partiels,
le courant partiel 1 (A, AA), ayant une température d'environ 40°C, est utilisé sans traitement pour l'empâtage d'une nouvelle biomasse,
tandis que le courant partiel 2 (B) est soumis à une préparation de substrat (10) en étant chauffé à environ 70 à 90°C,
on en soutire l'ammoniac/l'azote ammoniacal dans une opération d'épuisement,
la chaleur nécessaire à l'opération d'épuisement provenant de l'installation de cogénération KWK (20),
l'opération d'épuisement en elle-même ayant lieu à environ 75 à 90°C,
le résidu de fermentation amené et évacué est refroidi ou préchauffé dans le cadre d'un procédé à contre-courant (11),
le courant partiel 2, après l'opération d'épuisement, est envoyé en continu à un séparateur (13), par l'intermédiaire d'un échangeur de chaleur (11) à une température d'environ 45°C, sous forme d'un résidu de fermentation qui est maintenant hygiénisé et a une faible teneur en azote, par l'intermédiaire d'un réservoir tampon (12), et est séparé en une phase solide et une phase liquide,
la phase liquide est envoyée sous forme d'un recirculat (BB) au mélangeur (4),
la phase solide est séchée (14), ou directement utilisée sous forme d'une suspension, sous forme d'un engrais en agriculture.

2. Procédé pour l'utilisation de la biomasse dans un procédé de production de biogaz,
dans lequel d'abord une biomasse, présente sous forme d'une matière solide, est broyée dans un broyeur, empâtée, on y inocule des bactéries, et on soutire le biogaz formé,
où
la biomasse broyée, avec le recirculat, est empâtée dans un mélangeur, et le substrat est converti pour prendre un état apte au pompage,
puis le mélange apte au pompage est soumis à une homogénéisation plus poussée dans un broyeur en ligne, on procède à une hydrolyse et à une formation d'acide sous l'action de bactéries dans un ou plusieurs séparateurs montés en aval et munis d'un agitateur,
les constituants inorganiques sont séparés et jetés,
on procède dans des fermenteurs, en présence d'un mélange continu à haute température, à la formation d'acide acétique et à une méthanogenèse, avec formation d'un biogaz,
le biogaz est soutiré, éventuellement stocké, et envoyé à une utilisation énergétique,
tandis que le résidu de fermentation qui se forme est subdivisé en deux courants partiels, le courant partiel 1 restant non traité pour empâtage de la nouvelle biomasse, tandis que le courant partiel 2 est soumis à une préparation de substrat, par le fait qu'on en soutire, dans une opération d'épuisement, de l'ammoniac/de l'azote ammoniacal,
le courant partiel 2 est, après l'opération d'épuisement, éventuellement chauffé à l'aide d'un caloporteur, et maintenant, sous forme d'un résidu de fermentation hygiénisé et à faible teneur en azote, est envoyé en continu dans un séparateur par l'intermédiaire d'un récipient tampon, et est séparé en une phase solide et une phase liquide,
la phase liquide est envoyée au mélange sous forme de recirculat,
la phase solide est séchée, ou valorisée sous forme d'une suspension,
**caractérisé en ce que**, d'une manière correspondant à la Figure 3, la biomasse broyée, avec le recirculat, est empâtée dans un mélangeur (4), et le substrat est converti pour prendre un état apte au pompage, puis le mélange apte au pompage est soumis à une homogénéisation plus poussée dans un broyeur en ligne (5),
on procède, lors d'une ou plusieurs étapes préalables (7) montées en aval les unes des autres, avec agitateur central, à une hydrolyse et à une formation d'acide sous l'action de bactéries,
la suspension obtenue est ensuite soumise à une préparation de substrat (10) par chauffage à 70 à 90°C, on en soutire dans une opération d'épuisement l'ammoniac/l'azote ammoniacal, la chaleur nécessaire à l'opération d'épuisement provenant de l'installation de cogénération KWK (20),
l'opération d'épuisement a lieu à 70 à 90°C,
le résidu de fermentation amené et évacué est refroidi ou préchauffé dans un procédé à contre-courant (11),
dans un séparateur (23), les constituants inorganiques sont séparés et jetés,
dans des fermenteurs (8), en présence d'un mélange intime continu à une température de 30 à 60°C, on procède à une formation d'acide acétique et à une méthanogénèse,
le biogaz est soutiré, stocké (9) et envoyé à une utilisation énergétique,
le résidu de fermentation qui est maintenant hygiénisé et a une faible teneur en azote est envoyé en continu à un séparateur (13) et est séparé en une phase solide et une phase liquide,
la phase liquide est envoyée sous forme d'un recirculat au mélangeur (4),
la phase solide est séchée (14), ou directement utilisée sous forme d'une suspension.

3. Procédé selon l'une ou plusieurs des revendications 1 et 2, **caractérisé en ce que**, lors du lancement du procédé continu de production d'un biogaz, on utilise pour l'empâtage, au lieu du résidu de fermentation, de l'eau ou du lisier.

4. Procédé selon l'une ou plusieurs des revendications 1 à 3, **caractérisé en ce que** le temps de séjour dans l'étape préalable est de un à six jours.

5. Procédé selon l'une ou plusieurs des revendications 3 et 4, **caractérisé en ce que**, avec le courant partiel 1, le substrat subit une inoculation par des bactéries dans le mélangeur (4) ou dans le fermenteur (8).

6. Procédé selon l'une ou plusieurs des revendications 1 à 5, **caractérisé en ce que** la fermentation dans le fermenteur (8) a lieu dans des conditions mésophiles à des températures de 35 à 42°C ou thermophiles à des températures de 50 à 60°C.

7. Procédé selon l'une ou plusieurs des revendications 1 à 6, **caractérisé en ce que**, dans plusieurs fermenteurs (8), indépendamment les unes des autres on travaille dans des conditions mésophiles et thermophiles, et le temps de séjour dans les fermenteurs (8) est de 20 à 40 jours.

8. Procédé selon l'une ou plusieurs des revendications 1 à 7, **caractérisé en ce que** l'opération d'épuisement se déroule à une température > 70°C, pour une durée > 1 heure, sous forme d'un procédé discontinu.

9. Procédé selon l'une ou plusieurs des revendications 1 à 8, **caractérisé en ce que** l'opération d'épuisement se déroule, pour ce qui est de l'amenée et de l'évacuation des résidus de fermentation, dans un procédé thermique à contre-courant.

10. Procédé selon l'une ou plusieurs des revendications 1 à 9, **caractérisé en ce que**, de trois récipients d'épuisement, il y en a un qui est rempli, un qui est exploité et un qui est vidé, ou encore un qui est vidé et deux qui sont exploités ; de quatre récipients d'épuisement, il y en a un qui est rempli, un qui est vidé et deux qui sont exploités, le récipient tampon étant absent.

11. Procédé selon l'une ou plusieurs des revendications 1 à 10, **caractérisé en ce que** l'étape préalable (7) et les fermenteurs (8) sont chauffés à l'aide de la chaleur résiduelle de l'opération d'épuisement, et la phase solide est séchée dans l'entrepôt des résidus de fermentation (15).

12. Procédé selon l'une ou plusieurs des revendications 1 à 11, **caractérisé en ce qu'**on utilise en tant que biomasse à teneur élevée en substances étrangères du fumier de volaille, on envoie directement ce dernier à l'étape préalable (7), on y procède à un mélange intime, puis on envoie le mélange au broyeur en ligne.
